Europäisches Patentamt

European Patent Office

Office européen des brevets

(11) Numéro de publication: **0 220 352**
**A1**

## (12) DEMANDE DE BREVET EUROPEEN

(21) Numéro de dépôt: **85402015.3**

(22) Date de dépôt: **17.10.85**

(51) Int. Cl.4: **C01B 13/00 , B01J 19/08**

(43) Date de publication de la demande:
**06.05.87 Bulletin 87/19**

(84) Etats contractants désignés:
**AT BE CH DE GB IT LI LU NL SE**

(71) Demandeur: **Verpoorten, Georges Maurice**
**Résidence "Chanterelle" 92 bis, Av. Delattre De Tassigny**
**F-06400 Cannes(FR)**

(72) Inventeur: **Verpoorten, Georges Maurice**
**Résidence "Chanterelle" 92 bis, Av. Delattre De Tassigny**
**F-06400 Cannes(FR)**

(54) **Générateur d'oxonium par photocatalyse d'huiles essentielles de la famille des terpènes.**

(57) Il comprend un surpresseur 1,une cuve de barbotage 2,dans l'essence de térébenthine 3,une tubulure 4 pour débarrasser l'air des gouttelettes dans la cuve 5,vers le fond 6;l'air s'échappe en 7 dans la chambre 8,presse autour des sources de photons 9,et s'échappe en10.

L'invention peut être utilisée dans tous les cas où l'on cherche à recréer l'air pur des montagnes enrichi naturellement par la présence des résineux et les radiations solaires.

Fig. 1

EP 0 220 352 A1

## "GENERATEUR D'OXONIUM PAR PHOTO CATALYSE DES TERPENES".

L'objet de la présente invention est un générateur d'Oxonium par photocatalyse des terpènes.

On apprécie depuis toujours l'atmosphère règnant autour des résineux.Cela est du au dégagement des divers produits volatils des arbres de nos montagnes,comme les pins,les sapins et les mélèzes,parmi d'autres encore,dans l'air baigné de lumière riche en radiations solaires.Ceci explique la présence de nombreux centres de soins des voies respiratoires,en altitude.

Il est possible de recréer une atmosphère comparable à celle des stations telles qu'il en existe dans les Alpes,par exemple, mais en un lieu quelconque et dans un espace de l'ordre de celui d'une pièce,en y diffusant un produit tel que l'Oxonium.

L'objet de l'invention est donc de produire en tout lieu la photocatalyse qui se produit incessamment dans la nature depuis des millions d'années,en fournissant l'Oxonium.On pallie de la sorte tous les inconvénients des autres générateurs en reproduisant ce qui existe dans nos montagnes:l'air pur et la lumière solaire particulièrement riche en radiations.Quant à la source de produits résineux,on utilisera l'essence de térébenthine. Pour réaliser l'invention,on disposera d'un surpresseur débitant l'air ambiant dans une cuve fermée contenant de l'essence de térébenthine,par le moyen d'un tube qui y plonge pour permettre le barbotage. A la suite,un flacon retient les gouttelettes éventuellement entrainées.Une tubulure conduira l'air enrichi de vapeurs dans une chambre contenant des sources lumineuses dont la géométrie devra permettre d'occuper le plus possibble qu'il se pourra du volume de la chambre,dont les parois seront telles que le volume d'air se trouve laminé.Une tubulure d'échappement de l'air chargé d'Oxonium permet de le diriger vers la zône d'utilisation.

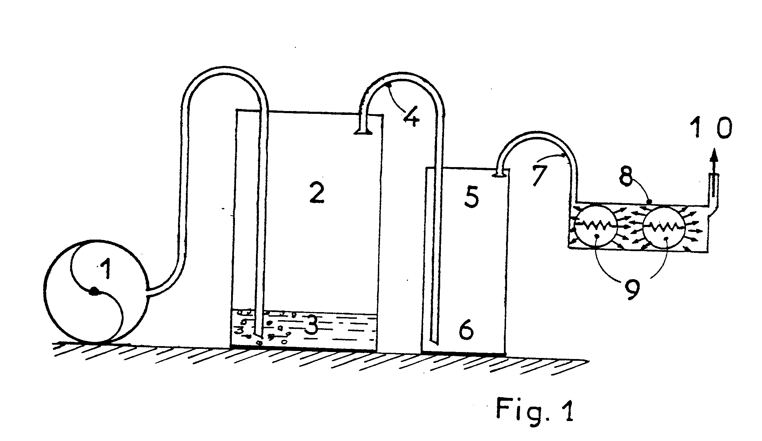

Le dessin annexé illustre,à titre d'exemple,un mode de réalisation du dispositif conforme à l'invention: 1 est le surpresseur, 2 la cuve de barbotage de l'air en partie basse dans la solution térébenthine repérée 3.La tubulure 4 conduit l'air enrichi dans la cuve 5 où la tubulure le conduit vers la base 6 pour perdre les gouttelettes qui pourraient avoir été entrainées.Un tube supérieur 7 conduit les vapeurs dans un espace 8 contenant les sources de photons dont la fonction peut être assimilée à celle d' Canon à Photons.Le volume de l'espace8 sera étudié afin que le cheminement de l'air,chargé de vapeurs terpèniques se fasse en régime laminaire contre les sources 9.

Les vapeurs d'air irradié s'échapperont en10,pour toute utilisation.

Le dispositif objet de l'invention peut être utilisé dans tous les cas où l'on désire créer une atmosphère riche en produits naturels que l'on rencontre à la campagne ou en montagne dans les forêts de résineux.Des inhalations de courte durée vaudront des séjours importants en montagne.

## Revendications

1-Générateur d'Oxonium par photocatalyse d'huiles essentielles de la famille des Terpènes,telle que l'essence de térébenthine, caractérisé par le fait qu'il consiste à faire traverser une faible épaisseur de vapeurs terpèniques par des rayons lumineux provoquant la photocatalyse.

2-Générateur suivant la revendication 1,caractérisé par le fait que le générateur de vapeurs est un petit surpresseur [1] injectant de l'air dans la partie basse d'une cuve [2] contenant un terpène.

3-Générateur suivant les revendications 1 et 2,caractérisé par le fait que l'air chargé de vapeurs terpèniques est débarrassé des gouttelettes dans une chambre de détente [5].

4-Générateur suivant les revendications 1,2 et 3,caractérisé par le fait que l'air chargé de vapeurs passe dans une chambre [8]où il est obligé de baigner,sous faible épaisseur,la totalité du corps d'une source de photons,avant de s'échapper par une tubulure [10], qui peut être orientable,dans l'atmosphère où on l'utilise

Fig. 1

## DOCUMENTS CONSIDERES COMME PERTINENTS

| Catégorie | Citation du document avec indication, en cas de besoin, des parties pertinentes | Revendication concernée | CLASSEMENT DE LA DEMANDE (Int. Cl.4) |
|---|---|---|---|
| A | BE-A- 780 645 (VANDENHEUVEL et al.)<br>* revendications 6, 7 * | 1 | C 01 B  13/00<br>B 01 J  19/08 |
| | --- | | |
| A | US-A-3 250 915 (SCHMITT et al.) | | |
| | --- | | |
| A | US-A-4 167 484 (MORIKAWA) | | |
| | ----- | | |

|  |  |  | DOMAINES TECHNIQUES RECHERCHES (Int. Cl.4) |
|---|---|---|---|
| | | | C 01 B  13/00<br>B 01 J  19/08 |

Le présent rapport de recherche a été établi pour toutes les revendications

| Lieu de la recherche | Date d'achèvement de la recherche | Examinateur |
|---|---|---|
| BERLIN | 09-06-1986 | CLEMENT J.P. |

CATEGORIE DES DOCUMENTS CITES

X : particulièrement pertinent à lui seul
Y : particulièrement pertinent en combinaison avec un autre document de la même catégorie
A : arrière-plan technologique
O : divulgation non-écrite
P : document intercalaire

T : théorie ou principe à la base de l'invention
E : document de brevet antérieur, mais publié à la date de dépôt ou après cette date
D : cité dans la demande
L : cité pour d'autres raisons

& : membre de la même famille, document correspondant

OEB Form 1503 03 82